(19) **Europäisches Patentamt European Patent Office Office européen des brevets**

(11) **EP 1 198 562 B1**

(12) **EUROPEAN PATENT SPECIFICATION**

| | |
|---|---|
| (45) Date of publication and mention of the grant of the patent:<br>**11.09.2013 Bulletin 2013/37** | (51) Int Cl.:<br>***C12N 9/98*** (2006.01)  ***C11D 3/386*** (2006.01)<br>***A23K 1/165*** (2006.01) |
| (21) Application number: **00943685.8** | (86) International application number:<br>**PCT/DK2000/000366** |
| (22) Date of filing: **07.07.2000** | (87) International publication number:<br>**WO 2001/004279 (18.01.2001 Gazette 2001/03)** |

(54) **A PROCESS FOR PREPARING AN ENZYME CONTAINING GRANULE**

VERFAHREN ZUR HERSTELLUNG EINES EIN ENZYM ENTHALTENDES GRANULAT

PROCEDE PERMETTANT DE PREPARER UN ENZYME CONTENANT UN GRANULE

| | |
|---|---|
| (84) Designated Contracting States:<br>**AT BE CH CY DE DK ES FI FR GB GR IE IT LI LU MC NL PT SE** | (72) Inventors:<br>• **MARCUSSEN, Erik**<br>  **DK-2750 Ballerup (DK)**<br>• **PEDERSEN, Christian**<br>  **DK-2610 Rødovre (DK)** |
| (30) Priority: **09.07.1999 DK 100099** | |
| (43) Date of publication of application:<br>**24.04.2002 Bulletin 2002/17** | (56) References cited:<br>**EP-A2- 0 304 331    EP-A2- 0 304 332**<br>**WO-A1-90/09428    WO-A1-90/09440**<br>**US-A- 4 740 469** |
| (73) Proprietor: **Novozymes A/S**<br>**2880 Bagsvaerd (DK)** | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to an improved process for preparing a dry granulated enzyme preparation and to products obtainable by such a process. The granulated enzyme preparation has through a controllable granule size an extended applicability and may show advantageous properties in a number of industrial applications such as incorporation of the granule in a feed or a baking composition.

BACKGROUND OF THE INVENTION

The industrial use of enzymes, notably enzymes of microbial origin, has become increasingly common. Enzymes are used in numerous industries, including, for example, the starch-processing industry, the food/feed industry and the detergent industry.

Since the introduction of enzymes into industrial applications a lot of effort has been devoted to improving the quality of enzyme products e.g. by applying one of a variety of known technologies for formulating a dry enzyme preparation each having their special characteristics. Such formulations may improve a range of quality parameters important to the value of the enzyme preparation. Parameters such as dust properties, solubility, enzyme stability per se, enzyme stability in a matrix, flow properties, color, odor, enzyme protein purity, particle size distribution, homogeneity, bulk density and pelleting stability are important quality parameters for dry enzyme products.

Known formulation technologies includes

- Spray dried products, wherein a liquid enzyme containing solution is atomized in a spray drying tower to form small droplets which during its way down the drying tower dries up to form an enzyme containing particulate material. Very small particles can be produced this way (Michael S. Showell (editor); Powdered detergents; Surfactant Science Series; 1998; vol. 71; page 140-142; Marcel Dekker).

- Layered products, wherein the enzyme is coated as a layer around a preformed core particle, wherein an enzyme containing solution is atomized, typically in a fluid bed apparatus wherein the preformed core particles are fluidized, and the enzyme containing solution adheres to the core particles and dries up to leave a layer of dry enzyme on the surface of the core particle. Particles of a desired size can be obtained this way if a useful core particle of the desired size can be found. This type of product is described in eg WO 97/23606

- Another type of product is known wherein an absorbing core particle is applied, and rather than coating the enzyme as a layer around the core, the enzyme is absorbed onto and/or into the surface of the core. Such a process is described in WO 97/39116.

- Extrusion or pelletized products, wherein an enzyme containing paste is pressed to pellets or under pressure is extruded through a small opening and cut into particles which is subsequently dried. such particles usually have a considerable size because of the material in which the extrusion opening is made (usually a plate with bore holes) sets a limit on the allowable pressure drop over the extrusion opening. Also very high extrusion pressures when using a small opening increases heat generation in the enzyme paste which is harmful to the enzyme. (Michael S. Showell (editor); Powdered detergents; Surfactant Science Series; 1998; vol. 71; page 140-142; Marcel Dekker)

- Prilled products, wherein an enzyme powder is suspended in molten wax and the suspension is sprayed, eg through a rotating disk atomizer, into a cooling chamber where the droplets quickly solidify (Michael S. Showell (editor); Powdered detergents; Surfactant Science Series; 1998; vol. 71; page 140-142; Marcel Dekker).

- Mixer granulation products, wherein an enzyme containing liquid is added to a dry powder composition of conventional granulating components. The liquid and the powder in a suitable proportion is mixed in and as the moisture of the liquid is absorbed in the dry powder, the components of the dry powder will start to adhere and agglomerate and particles will build up forming granules comprising the enzyme. Such a process is described in 4,106,991 (NOVO NORDISK) and related documents EP 170360 B1 (NOVO NORDISK), EP 304332 B1 (NOVO NORDISK), EP 304331 (NOVO NORDISK), WO 90/09440 (NOVO NORDISK) and WO 90/09428 (NOVO NORDISK). The invention described herein, preferably belongs to this class of dry granulated enzyme preparations.

In many applications for such enzyme products an important property of an enzyme granule is the size and/or the size distribution of the granule as most frequently the application of an enzyme granule involves mixing the granules with other dry products, e.g. detergent, fodder or flour compositions. Use of an enzyme granule of a proper size in such compositions may provide a more homogeneous distribution of the enzyme granule in the composition and a less tendency of the enzyme granules separating from the other composition components when stored in such compositions. If the enzyme granules do not possess the proper size distribution compared to the composition in which they are used it is often observed that the enzyme granule upon prolonged storage or handling of the composition concentrates in specific parts or layers of the composition, e.g. near the bottom or top of the container holding the composition. However, manufacture of enzyme granules are delicate often empirical processes designed to provide the granules with a range of desired properties as mentioned above, and usually a desired size of an enzyme granule may not be chosen at will, without changing and/or deteriorating other desired properties of the granules. Typically in a granulation process granules having a wide size distribution range are produced e.g. from 100 $\mu$m to 2000 $\mu$m which is undesirable, as there may be significant difference in properties of between smaller granules compared to larger granules. Of course it may be possible to obtain a specific size fraction of an enzyme granule by sieving the product to a proper size, but this is an undesired process as product outside the desired size range would have to be discarded or reprocessed. It would be much more advantageous if the product directly obtainable from the process possessed a desired size and size distribution.

In the previously mentioned disclosures concerning mixer granulation products the granules are described having a mean diameter preferably between 2 and 1000 $\mu$m. However the lowest mean granule size is achieved in practice is 390 $\mu$m (Example 1 in EP 304332 B1) and where more than 77% of the granules have a size above 300 $\mu$m. Also WO 98/54980 (GIST BROCADES) describes an enzyme containing granule comprising an edible carbohydrate polymer and water having a size between 100 and 2000 $\mu$m. Again however, all exemplified granules have an mean particle diameter of at least 480 $\mu$m. These granule is further described as free of soap, detergents and bleach or bleach compounds, zeolites, binders and fillers, such as $TiO_2$, kaolin, silicates talc etc.

Other relevant documents are EP 321481 B1 (GIST BROCADES) which describes microgranules composed of a biological material e.g. an enzyme immobilized in a gelled or gelable material such as k-carragenan, alginic acid, cellulose or its derivatives. The micro granule which has a preferred size of 50-500 $\mu$m is coated with a coating which is insoluble at acidic solutions but soluble in alkali or intestinal solutions. EP 257 996 B1 (CULTOR) relates to an enzyme premix composition comprising a grain carrier such as wheat flour and less than 10% water. This is however not a granulated product. EP 168 526 B1 (HENKEL) discloses enzyme containing granules of a size between 100-2000 $\mu$m wherein the amount of granules below 100 $\mu$m constitutes less than 0.2% w/w of the granules. The granules comprises besides from protease/amylase also crystalline zeolite, a water swellable starch, carboxymethylcellulose or polyethyleneglycols and inorganic salts. WO 92/11347 (HENKEL) describes an enzyme containing granule prepared by extrusion of a size between 100-2000 $\mu$m comprising enzyme, a water swellabel starch, granulating agents, a water soluble polymer, salts, water and cereal flour. WO 97/42837 (HOECHST) discloses an enzyme containing granule prepared by high speed mixing comprising enzyme, granulating agents and a cereal flour. Granules prepared from this process have a preferred size of 50-800 $\mu$m, as granules below 50 $\mu$m and above 800 $\mu$m are removed in a fluid bed dryer. Further WO 97/43482 (GENENCOR) and WO 97/42839 (GENENCOR) describes a method for producing a high shear mixer granule having 75-99.9 parts of a super heated steam treated organic flour with a grinding degree of 30-100% as well as enzyme and optionally auxiliary granulation agents.

SUMMARY OF THE INVENTION

Within the field of mixer granulation technology, we have developed a process providing an improved control of the size and/or size distribution of the resulting enzyme containing mixer granulation products. By adding a particulate component of a suitable size having a diameter less than the diameter of the finished granule, to a conventional mixer granulation process (i.e. incorporating enzymes and conventional granulating components), granules may be build for which the size and/or size distribution will be at least partially depending or controlled of the size and properties of the added particulate component. Accordingly in a first aspect the invention provides:

A process for manufacture of a dry enzyme containing mixer granulation granule comprising the step of adding a particulate component to the mixer granulation process, wherein the particulate component constitutes less than 75 parts of the finished granule and the particles of the particulate component have an mean size of more than 40 $\mu$m in its longest dimension.

In accordance with this first aspect a second aspect of the invention relates to a granular enzyme product obtainable from the above mentioned process and to enzyme containing granules containing at least two particles of the particulate component of the first aspect.

In further aspects the invention provides

- a feed composition comprising the above mentioned granular enzyme product.
- a flour and/or baking and/or dough composition comprising the above mentioned granular enzyme product.
- a detergent composition comprising the above mentioned granular enzyme product.

And to methods of their use.

DESCRIPTION OF DRAWING

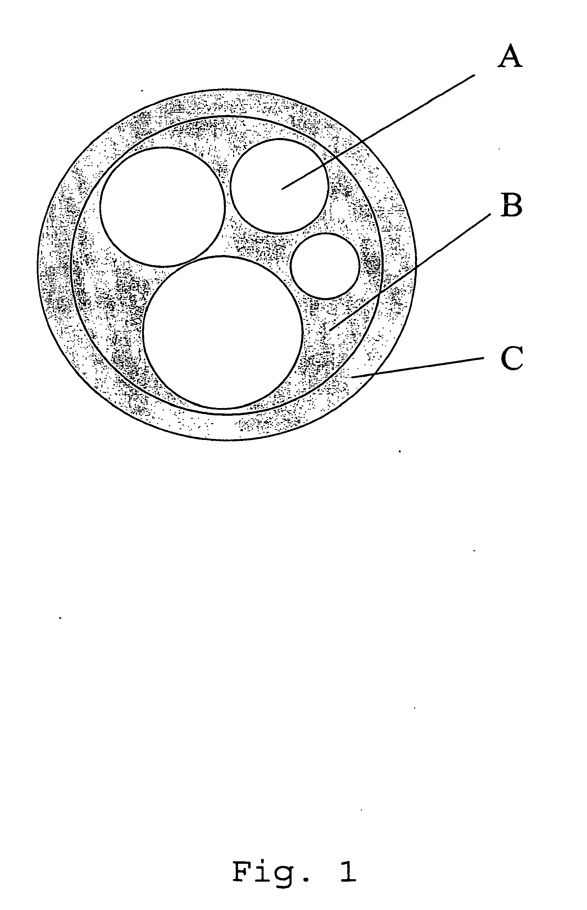

Figure 1 depicts an example of a granule of the invention. In the figure A is the particulate component, B is the conventional granulation components including enzymes and C is an optional coating.

DETAILED DESCRIPTION OF THE INVENTION

As described *vide supra* we have developed a mixer granulation process providing an improved control of the size and/or size distribution of the resulting enzyme containing mixer granulation products (granules). By adding a particulate component of a suitable size having a diameter less than the diameter of the final granule, to a conventional mixer granulation process (i.e. incorporating enzymes and conventional granulating components), granules may be build which size and/or size distribution will be at least partially depending or controlled of the size and properties of the added particulate component. An advantage of the present invention is that the improved control of the size of a finished mixer granule as provided by the process of the invention lowers the need for further processing of the granule such as sieving, separation and/or recirculation of odd-sized T-granules. Also the process of the invention provides for the manufacture of granules having a lower mean size than may be achieved using the conventional mixer granulation processes known to the art.

**Definitions**

It is to be understood that the term "mixer granulation" represents a granulation technology, wherein a liquid is added to a dry powder composition and by mixing these components granules are build by agglomeration of the solid in the powder and the liquid. The mixer equipment is conventional mixer equipment such as high shear mixers or intensive high shear mixers or all other mixer types known to the art. The mixing equipment can be a batch mixer or a continuous mixer, such as a convective mixer [see, e.g., Harnby et al., Mixing in the Process Industries, pp. 39- 53 (ISBN 0- 408- 11574- 2) ] . Non- convective mixing equipment, e.g. rotating drum mixers or so- called pan- granulators, may also be employed.
It is to be understood that the term "size" of particles or granules covers the diameter of a particle or granule measured in the longest dimension of the particle or granule. Also the "mean size" of particles or granules is to be understood as the mean diameter of the particles or granules measured in the longest dimension of the particles or granules. The terms "particles" and "granules" are to be understood as a predominantly spherical or near spherical structure of a macromolecular size as opposed to the term "fiber" which is to be understood as a rod or thread like macromolecular structure having a dimension wherein the length of the structure greatly exceeds the width of the structure. The spherical particles should preferably have a ratio, (a):(b), between the diameter in the shortest dimension (a) and the diameter in the longest dimension (b) of the particle of between 1:1 to 1:5, preferably between 1:1 to 1:3.
It is to be understood that the term "conventional granulating components" covers solids of a mean size which is less than 40 $\mu$m in its longest dimension, optionally in combination with fibers such as cellulose fibers. Usually these powders are solids which have been finely ground to small particle size to achieve properties of the powder suitable for a conventional mixer granulation process.
The particle or granule "size distribution" (PSD) can be expressed in terms of the mass mean diameter of the individual particles. A mean mass diameter of D50 is the diameter at which 50% of the granules, by mass, have a smaller diameter, while 50% by mass have a larger diameter. The values D10 and D90 are the diameters at which 10% and 90%, respectively, of the granules, by mass, have a smaller diameter than the value in question. The "span" indicates the breadth of the PSD and is expressed as:

$$(D90 - D10) / D50.$$

For purposes of the present invention, the particle or granule size distribution is normally as narrow as possible. The span of a granulate product according to the invention is therefore typically less than about 2.5, preferably less than about 2.0, more preferably less than about 1.5, and most preferably less than about 1.0.

**The particulate component**

The particulate component of the invention may be any particulate material suitable for mixer granulation. Of course as described, *infra,* conventional granulating components used for building of the granule are usually also in a particulate form, but in order to achieve granules of a size desired for many applications, the particulate component should have an mean size which is significantly larger than the particles of the conventional granulating components and yet smaller than the size of the finished granule. Applying such particles enables a mixer granulation process in which a low mean granule size of the finished granules may be obtained. Accordingly the build up of granules is thought to be comparable with a "brick and mortar" type of construction, wherein the particles of the particulate component constitutes the bricks and the conventional granulating components the mortar.

The mean particle size of the particulate component is as said, *supra,* at least 40 $\mu$m, more preferred at least 60 $\mu$m such as at least 80 $\mu$m, e.g. at least 100 $\mu$m. Some useful particulate components may be even larger e.g. have a mean particle size of at least 140 $\mu$m or even at least 200 $\mu$m. Depending on the desired upper size limit of the finished granule the particulate component may have any mean size of more than 40 $\mu$m as long as it is smaller than the desired mean size of the finished mixer granule.

In a preferred embodiment, the particulate component has a size, which enables that the finished granule contains at least two particles of the particulate component having a mean size of more than 40 $\mu$m in its longest dimension, and preferably the mean diameter of the particulate component in its longest dimension is less than half the mean diameter of the finished granule in its longest diameter. More preferably the finished granule contains more than three, more than four, more than five or more than six particles of the particulate component such as 3-15 particles of the particulate component

In order to provide an even further improved control of the mean size of the finished granule it may be desired to use a particulate component which have a narrow particle size distribution. Accordingly it is preferred to use a particulate component for which at least 80% w/w of the particles have a size within the range of plus or minus 40%, e.g. plus or minus 30% or plus or minus 20% of the mean size of the particulate component. Most preferred the particulate component meet the above mentioned requirements of SPAN values for the finished granules. i.e. having a SPAN value of less than about 2.5, preferably less than about 2.0, more preferably less than about 1.5, and most preferably less than about 1.0. It is expedient in the method according to the invention to use vegetable flours that are obtained by grinding of cereal grains. The cereals that can serve as flour sources within the scope of the invention are especially wheat or rye, but barley, oats, rice and maize, as well as sorghum and other types of millet can also be used. Although buckwheat itself is not a cereal (it is a knot grass), its beechnut-like flour-yielding parts can likewise be used as flour source within the scope of the invention. In a particular variation of the invention legumes may serve as a flour source. A preferred particulate compound is a wheat based flour such as the commercially available product Farigel ((Farigel de Ble F1100, WestHove, France). The vegetable flour of the invention has preferably been subjected to a steam treatment e.g. with dry superheated steam with a temperature of about 100°C to about 110°C at nearly normal pressure to low over pressure (e.g., 0.8 to 1.2 bar over pressure) and a treatment time (residence time in the superheated steam treatment apparatus described below) of up to about 1 hour. Dry superheated steam is a superheated and unsaturated steam, which can be obtained in the conventional way by superheating and removal of possible water condensate or by expansion of steam from high pressure. The particulate component of the invention is distinguished by the steam treatment being done after grinding the vegetable flour source, i.e. on the prepared particulate component ready to be used in the mixer granulation process. The advantages of using a steam treated particulate component is of course that that it lowers the number of bacteria or fungus present in the particulate component which may cause microbial growth in the product, but more important the particulate component will be fully or partly gelatinised. Gelatinising improves the integrity of the particles so they do not disintegrate, dissolves or becomes dispersed in the granulation process, but keep their particulate characteristics. Steam treatment of the particulate compound may e.g. take place while using a conical hopper that becomes wider toward the bottom, which is equipped with one or more ring nozzles for steam lances for introduction of the dry superheated steam. The hopper may intermittently or continuously be supplied with the flour source, e.g. through screw conveyors and evacuated through heated screw conveyors.

**The conventional granulating components**

The conventional granulating components as mentioned, *supra,* are components known as useful for the manufacture of conventional mixer granulation products, e.g. as described in US 4,106,991, which is hereby incorporated by reference. As said, *supra,* the build up of granules of the invention is thought to be comparable with a "brick and mortar" type of construction wherein the conventional granulating components is the mortar. The conventional granulating components may include but is not limited to:

a) Fillers such as fillers conventionally used in the field of granulation e.g. water soluble and/or insoluble inorganic salts such as finely ground alkali sulphate, alkali carbonate and/or alkali chloride), clays such as kaolin (e.g. Speswhite™, English China Clay), bentonites, talcs, zeolites, and/or silicates.

**b)** Binders such as binders conventionally used in the field of granulation e.g. binders with a high melting point or no melting point at all and of a non waxy nature e.g. polyvinyl pyrrolidon, dextrins, polyvinylalkohol, cellulose derivatives, for example hydroxypropyl cellulose, methyl cellulose or CMC. A suitable binder is a carbohydrate binder such as Glucidex 21D available from Roquette Freres, France.

**c)** Fiber materials such as fibers conventionally used in the field of granulation. Pure or impure cellulose in fibrous form can be sawdust, pure fibrous cellulose, cotton, or other forms of pure or impure fibrous cellulose. Also, filter aids based on fibrous cellulose can be used. Several brands of cellulose in fibrous form are on the market, e.g. CEPO and ARBOCELL. In a publication from Svenska Tramjolsfabrikerna AB, "Cepo Cellulose Powder" it is stated that for Cepo S/20 cellulose the approximate maximum fiber length is 500 $\mu$m, the approximate average fibre length is 160 $\mu$m, the approximate maximum fibre width is 50 $\mu$m and the approximate average fibre width is 30 $\mu$m. Also, it is stated that CEPO SS/200 cellulose has an approximate maximum fibre length of 150 $\mu$m, an approximate average fibre length of 50 $\mu$m, an approximate maximum fiber width of 45 $\mu$m and an approximate average fiber width of 25 $\mu$m. Cellulose fibers with these dimensions are very well suited for the purpose of the invention. The words "Cepo" and "Arbocel" are Trade marks. A preferred fibrous cellulose is Arbocel™ BFC200. Also synthetic fibres may be used as described in EP 304331 B1 and typical fibres may be made of polyethylene, polypropylene, polyester, especially nylon, polyvinylformat, poly(meth)acrylic compounds.

**d)** Liquid agents such as conventionally used in the field of granulation. A liquid agent is used in conventional mixer granulation processes for enabling the build up or agglomeration of the conventional granulating component particles into granules. The liquid agent is water and/or a waxy substance. The liquid agent is always used in a liquid phase in the granulation process but may later on solidify; the waxy substance if present, therefore, is either dissolved or dispersed in the water or melted. By the term "waxy substance" as used herein is meant a substance which possesses all of the following characteristics 1) the melting point is between 30 and 100°C, preferably between 40 and 60°C, 2) the substance is of a tough and not brittle nature, and 3) the substance possesses a certain plasticity at room temperature. Both water and waxy substance are liquid agents, i.e. they are both active during the formation of the granules; the waxy substance stays as a constituent in the finished granules, whereas the majority of the water is removed during a drying step. Examples of waxy substances are polyglycols, fatty alcohols, ethoxylated fatty alcohols, mono-, di- and triglycerolesters of higher fatty, acids, e.g. glycerol monostearate, alkylarylethoxylates, and coconut monoethanolamide.

If a high amount of waxy substance is used, relatively little water should be added, and vice versa. Thus, the liquid agent can be either water alone, waxy substance alone or a mixture of water and waxy substance. When a mixture of water and waxy substance is used, the water and the waxy substance can be added in any sequence, e.g. first the water and then the waxy substance, or first the waxy substance and then the water or a solution or suspension of the waxy substance in the water. Also, when a mixture of water and waxy substance is used, the waxy substance can be soluble or insoluble (but dispersible) in water. If water is used a liquid agent it may not be a part of the finished mixer granule as usually most of the water is dried off off at a subsequent drying of the mixer granules.

**e)** Enzyme stabilizing or protective agents such as conventionally used in the field of granulation. Stabilizing or protective agents may fall into several categories: alkaline or neutral materials, reducing agents, antioxidants and/or salts of first transition series metal ions. Each of these may be used in conjunction with other protective agents of the same or different categories. Examples of alkaline protective agents are alkali metal silicates, -carbonates or bicarbonates which provide a chemical scavenging effect by actively neutralizing e.g. oxidants. Examples of reducing protective agents are salts of sulfite, thiosulfite or thiosulfate, while examples of antioxidants are methionine, butylated hydroxytoluene (BHT) or butylated hydroxyanisol (BHA). Most preferred agents are salts of thiosulfates, e.g. sodium thiosulfate. Also enzyme stabilizers may be borates, borax, formates, di- and tricarboxylic acids and reversible enzyme inhibitors such as organic compounds with sulfhydryl groups or alkylated or arylated boric acids.

**f)** Crosslinking agents such as conventionally used in the field of granulation. Crosslinking agents may be enzyme-compatible surfactants eg ethoxylated alcohols, especially ones with 10 to 80 ethoxy groups.

Further suspension agents, mediators (for boosting bleach action upon dissolution of the granule in e.g. a washing application or mediators for enzymes) and/or solvents may be incorporated as conventional granulating agents.

**Enzymes**

The enzyme in the context of the present invention may be any enzyme or combination of different enzymes, which benefits from being granulated in order to be applicable for a specific use. Accordingly, when reference is made to "an enzyme" this will in general be understood to include combinations of one or more enzymes.

It is to be understood that enzyme variants (produced, for example, by recombinant techniques) are included within the meaning of the term "enzyme". Examples of such enzyme variants are disclosed, e.g., in EP 251, 446 (Genencor), WO 91/00345 (Novo Nordisk), EP 525, 610 (Solvay) and WO 94/02618 (Gist-Brocades NV).

The enzyme classification employed in the present specification with claims is in accordance with Recommendations (1992) of the Nomenclature Committee of the International Union of Biochemistry and Molecular Biology, Academic Press, Inc., 1992.

Accordingly the types of enzymes which may appropriately be incorporated in granules of the invention include oxidoreductases (EC 1.-.-.-), transferases (EC 2.-.-.-), hydrolases (EC 3.-.-.-), lyases (EC 4.-.-.-), isomerases (EC 5.-.-.-) and ligases (EC 6.-.-.-).

Preferred oxidoreductases in the context of the invention are peroxidases (EC 1.11.1), laccases (EC 1.10.3.2) and glucose oxidases (EC 1.1.3.4)], while preferred transferases are transferases in any of the following sub-classes:

   a) Transferases transferring one-carbon groups (EC 2.1);
   b) transferases transferring aldehyde or ketone residues (EC 2.2); acyltransferases (EC 2.3);
   c) glycosyltransferases (EC 2.4);
   d) transferases transferring alkyl or aryl groups, other that methyl groups (EC 2.5); and
   e) transferases transferring nitrogeneous groups (EC 2.6).

A most preferred type of transferase in the context of the invention is a transglutaminase (protein-glutamine $\gamma$-glutamyltransferase; EC 2.3.2.13).

Further examples of suitable transglutaminases are described in WO 96/06931 (Novo Nordisk A/S).

Preferred hydrolases in the context of the invention are: Carboxylic ester hydrolases (EC 3.1.1.-) such as lipases (EC 3.1.1.3); phytases (EC 3.1.3.-), e.g. 3-phytases (EC 3.1.3.8) and 6-phytases (EC 3.1.3.26); glycosidases (EC 3.2, which fall within a group denoted herein as "carbohydrases"), such as $\alpha$-amylases (EC 3.2.1.1); peptidases (EC 3.4, also known as proteases); and other carbonyl hydrolases].

In the present context, the term "carbohydrase" is used to denote not only enzymes capable of breaking down carbohydrate chains (e.g. starches) of especially five- and six-membered ring structures (i.e. glycosidases, EC 3.2), but also enzymes capable of isomerizing carbohydrates, e.g. six-membered ring structures such as D-glucose to five-membered ring structures such as D-fructose.

Carbohydrases of relevance include the following (EC numbers in parentheses):

   $\alpha$- amylases (3.2.1.1), $\beta$- amylases (3.2.1.2), glucan 1, 4- $\alpha$- glucosidases (3.2.1.3), cellulases (3.2.1.4), endo-1, 3 (4)- $\beta$- glucanases (3.2.1.6), endo- 1, 4- $\beta$- xylanases (3.2.1.8), dextranases (3.2.1.11), chitinases (3.2.1.14), polygalacturonases (3.2.1.15), lysozymes (3.2.1.17), $\beta$- glucosidases (3.2.1.21), $\alpha$- galactosidases (3.2.1.22), $\beta$- galactosidases (3.2.1.23), amylo- 1, 6- glucosidases (3.2.1.33), xylan 1, 4- $\beta$- xylosidases (3.2.1.37), glucan endo- 1, 3- $\beta$- D- glucosidases (3.2.1.39), $\alpha$- dextrin endo- 1, 6- $\alpha$- glucosidases (3.2.1.41), sucrose $\alpha$- glucosidases (3.2.1.48), glucan endo- 1, 3- $\alpha$- glucosidases (3.2.1.59), glucan 1, 4- $\beta$- glucosidases (3.2.1.74), glucan endo- 1, 6- $\beta$- glucosidases (3.2.1.75), arabinan endo- 1, 5- $\alpha$- L- arabinosidases (3.2.1.99), lactases (3.2.1.108), chitosanases (3.2.1.132) and xylose isomerases (5.3.1.5).

Examples of commercially available oxidoreductases (EC 1.-.-.-) include Gluzyme™ (enzyme available from Novo Nordisk A/S). Examples of commercially available proteases (peptidases) include Kannase™, Everlase™, Esperase™, Alcalase™, Neutrase™, Durazym™, Savinase™, Pyrase™, Pancreatic Trypsin NOVO (PTN), Bio-Feed™ Pro and Clear-Lens™ Pro (all available from Novo Nordisk A/S, Bagsvaerd, Denmark).

Other commercially available proteases include Maxatase™, Maxacal™, Maxapem™, Opticlean™ and Purafect™ (available from Genencor International Inc. or Gist-Brocades).

Examples of commercially available lipases include Lipoprime™ Lipolase™, Lipolase™ Ultra, Lipozyme™, Palatase™, Novozym™ 435 and Lecitase™ (all available from Novo Nordisk A/S).

Other commercially available lipases include Lumafast™ (*Pseudomonas mendocina* lipase from Genencor International Inc.); Lipomax™ (*Ps. pseudoalcaligenes* lipase from Gist-Brocades/Genencor Int. Inc.; and *Bacillus* sp. lipase from Solvay enzymes.

Examples of commercially available carbohydrases include Alpha-Gal™, Bio-Feed™ Alpha, Bio-Feed™ Beta, Bio-Feed™ Plus, Bio-Feed™ Plus, Novozyme™ 188, Celluclast™, Cellusoft™, Ceremyl™, Citrozym™, Denimax™,

Dezyme™, Dextrozyme™, Finizym™, Fungamyl™, Gamanase™, Glucanex™, Lactozym™, Maltogenase™, Pentopan™, Pectinex™, Promozyme™, Pulpzyme™, Novamyl™, Termamyl™, AMG™ (Amyloglucosidase Novo), Maltogenase™, Sweetzyme™ and Aquazym™ (all available from Novo Nordisk A/S). Further carbohydrases are available from other suppliers.

The amount of enzyme to be incorporated in a granule of the invention will depend on the intended use of the granulate. For many applications, the enzyme content will be as high as possible or practicable.

The content of enzyme (calculated as pure enzyme protein) in a granule of the invention will typically be in the range of from about 0.5% to 50% by weight of the enzyme-containing granule.

When, for example, a protease (peptidase) is incorporated in granules according to the invention, the enzyme activity (proteolytic activity) of the finished granules will typically be in the range of 1- 20 KNPU/g. This unit for protease activity is Kilo Novo Protease Units per gram of sample (KNPU/g) . The activity is determined relatively to an enzyme standard of known activity in KNPU/g. The enzyme standard is standardized by measuring for a given amount of enzyme the formation rate ($\mu$mol/ minute) of free amino groups liberated from digestion of di- methyl- casein (DMC) in solution by the enzyme. The formation rate is monitored by recording the linear development of absorbance at 420 nm of the simultaneous reaction between the formed free amino groups and added 2, 4, 6- tri- nitro- benzene- sulfonic acid (TNBS) . The digestion of DMC and the color reaction is carried out at 50°C in a pH 8.3 boric acid buffer with a 9 min. reaction time followed by a 3 min. measuring time. A folder AF 220/1 is available upon request to Novo Nordisk A/S, Denmark, which folder is hereby included by reference.

Likewise, in the case of, for example, $\alpha$- amylases, an activity of 10- 500 KNU/g will be typical. The activity is determined relatively to an enzyme standard of known activity in KNU/g. The enzyme standard is standardized by measuring for a given amount of enzyme the formation rate ($\mu$mol/ minute) of 2- chlor- 4- nitrophenol liberated from digestion of 2- chlor- 4- nitrophenyl- b- D- maltoheptaosid substrate by the enzyme and auxiliary alfa- and beta- glucosidase enzymes in solution. Kits for performing $\alpha$- amylase assays are commercially available. One description of an $\alpha$- amylase assay may be found in the leaflet AF318/1- GB available upon request from Novo Nordisk A/S, Denmark. For e.g. lipases, an activity in the range of 50- 400 KLU/g will normally be suitable.

Usually the enzyme will be applied to the granulation process as an enzyme containing liquid. The enzyme containing liquid may be applied as a purified product in which the enzyme is dissolved or dispersed as crystalline and/or amorphous protein in an aqueous liquid in the form of an enzyme concentrate or the enzyme containing liquid may be in the form of a fermentation broth. The water in the liquid may be used as a liquid agent for the granulation process (supra).

**The mixer granulation process**

The mixer granulation process of the invention may be conducted in an conventional manner, preferably a high shear mixing granulation process, such as described in US 4,106,991 e.g. Example 1, wherein conventional granulating components as a dry solid composition is brought in contact with a conventional liquid agent and mixed in a conventional granulation mixer as described supra, the amount of liquid being in such amount that granule will be formed or build up. The enzyme may either be present in dry form as a part of the dry solid composition or it may be contained in the liquid in a dissolved form or dispersed in the liquid as crystalline and/or amorphous protein particles. Such granule are in the art known e.g. as the so-called T-granules such as described in US 4,106,991. The invention is distinguished by including the particulate component of the invention in the process.

The particulate component of the invention may be added to the process either before addition of the liquid (added to a dry solid composition of the conventional granulating agents) , which is preferred or it may added during the mixing of the liquid and the other conventional granulating agents, but before granules have begun to build up (i.e. the particulate component may be added when the mixture of liquid and other conventional granulating components is in the form of a wetted powder. After the granules have been formed or build they are usually dried and optionally coated with a protective coating, e.g. by conventional methods in a fluid bed dryer. Drying and coating of the enzyme containing granule may be performed in any type of fluidising equipment (such as in a fluid- bed apparatus or other form of fluidising equipment, such as a Hüttlin- type fluidiser) . For a description of suitable fluid bed equipment, see, e.g., Harnby et al., Mixing in the Process Industries, pp. 54- 77 (ISBN 0- 408- 11574- 2) .

Accordingly the invention encompasses a process for the manufacture of an enzyme containing mixer granule comprising the steps of:

a) adding less than 75 of 100 parts of a particulate component having an mean size of more than 40 $\mu$m in its longest dimension to more than 25 of 100 parts of an enzyme and conventional granulating components and mixing these ingredients to form an enzyme containing granule,
b) drying the granules and
c) optionally coating the granules

Conventional coating methods may be used to apply the coating according to the invention as described by the references stated in the previous section (above).

The coating may preferably be applied by conventional methods in a mixer by mixing the uncoated granule with the coating materials. In another specific embodiment of the invention the coating is applied in a fluid bed process comprising:

a) fluidising an enzyme containing mixer granule in a fluid bed apparatus,

b) introducing a liquid medium comprising the coating material(s) by atomization of the liquid medium into the fluid bed, so as to deposit nonvolatile components of the liquid medium as a solid coating layer on the core material and,

c) removing volatile components of the liquid medium from the coated granule.

**Coatings**

The mixer granules obtained by the process of the invention may optionally, but preferably be coated with one or more coating layers to provide further improved properties of the granule. Conventional coatings as known to the art may suitably be used such as the coatings described in WO 89/08694, WO 89/08695, 270 608 B1 and/or PA 1998 00876 (Danish priority application unpublished at the priority date of this invention). Other examples of conventional coating materials may be found in US 4,106,991, EP 170360, EP 304332, EP 304331, EP 458849, EP 458845, WO 97/39116, WO 92/12645A, WO 89/08695, WO 89/08694, WO 87/07292, WO 91/06638, WO 92/13030, WO 93/07260, WO 93/07263, WO 96/38527, WO 96/16151, WO 97/23606, US 5,324,649, US 4,689,297, EP 206417, EP 193829, DE 4344215, DE 4322229 A, DD 263790, JP 61162185 A and/or JP 58179492.

In a particular embodiment the coating may comprise minor amounts of a protective agent capable of reacting with a component capable of inactivating (being hostile to) the enzyme entering the granule from a surrounding matrix, i.e. before the component come into contact and inactivate the enzyme. The protective agent may thus e.g. be capable of neutralizing, reducing or otherwise reacting with the component rendering it harmless to the enzyme. Typical components capable of inactivating the enzyme are oxidants such as perborates, percarbonates, organic peracids and the like.

Protective agents may fall into several categories: alkaline or neutral materials, reducing agents, antioxidants and/or salts of first transition series metal ions. Each of these may be used in conjunction with other protective agents of the same or different categories. Examples of alkaline protective agents are alkali metal silicates, -carbonates or bicarbonates which provide a chemical scavenging effect by actively neutralizing e.g. oxidants. Examples of reducing protective agents are salts of sulfite, thiosulfite or thiosulfate, while examples of antioxidants are methionine, butylated hydroxytoluene (BHT) or butylated hydroxyanisol (BHA). Most preferred agents are salts of thiosulfates, e.g. sodium thiosulfate. The amounts of protective agent in the coating may be 5-40% w/w of the coating, preferably 5-30%, e.g. 10-20%.

The coating should encapsulate the enzyme containing granule by forming a substantially continuous homogenous layer.

The coating may perform any of a number of functions in the granule, depending on the intended use of the granule. Thus, for example, a coating may achieve one or more of the following effects:

(i) further reduction of the dust-formation tendency of an enzyme granule;

(ii) further protection of enzyme(s) in the enzyme granule against oxidation by bleaching substances/systems (e.g. perborates, percarbonates, organic peracids and the like);

(iii) dissolution at a desired rate upon introduction of the granule into a liquid medium (such as an aqueous medium);

(iv) provide a better physical strength of the enzyme granule.

The coating may further comprise one or more of the following: anti-oxidants, chlorine scavengers, plasticizers, pigments, lubricants (such as surfactants or antistatic agents) additional enzymes and fragrances.

Plasticizers useful in coating layers in the context of the present invention include, for example: polyols such as sugars, sugar alcohols, or polyethylene glycols (PEGs) having a molecular weight less than 1000; urea, phthalate esters such as dibutyl or dimethyl phthalate; and water.

Suitable pigments include, but are not limited to, finely divided whiteners, such as titanium dioxide or kaolin, coloured pigments, water soluble colorants, as well as combinations of one or more pigments and water soluble colorants.

As used in the present context, the term "lubricant" refers to any agent which reduces surface friction, lubricates the surface of the granule, decreases tendency to build-up of static electricity, and/or reduces friability of the granules. Lubricants can also play a related role in improving the coating process, by reducing the tackiness of binders in the coating. Thus, lubricants can serve as anti-agglomeration agents and wetting agents.

Examples of suitable lubricants are polyethylene glycols (PEGs) and ethoxylated fatty alcohols.

In a preferred embodiment of the invention the granule of the invention is coated with a protective coating having a high constant humidity such as described in the Danish patent application PA 1998 00876 pages 5-9 and given examples which was unpublished at the date of filing this application and which is hereby incorporated by reference.

A finished granule whether it is coated or not may be provided by the process of the invention which preferably have a mean size of less than 390 $\mu$m when the granule is free of cellulose fibres and less than 480 $\mu$m when the granule contains cellulose fibres.

Also the invention provides an enzyme containing granule comprising at least two particles of a particulate component having a mean size of at least 40 $\mu$m, preferably wherein the mean diameter of the particulate component in its longest dimension is less than half the mean diameter of the finished granule in its longest diameter.

## Applications of the enzyme containing granule

The enzyme containing mixer granule according to the invention is useful where ever enzymes are to be stored alone or to be incorporated in another dry product, and an improved enzyme stability is needed to enable good storage and/or processability properties of the enzyme in the granule. The granule is for example useful in dry products comprising oxidative compounds such as peroxides or superoxides, e.g. bleach (e.g. perborates or percarbonates) or other reactive components, which in case of contact with the enzyme is able of inactivating the enzyme. Thus the invention provides a composition comprising the granule of the invention. The composition is preferably a detergent composition further comprising a surfactant The enzyme containing granule is further useful in a method for cleaning an object (e.g. a cellulose containing fabric such as textile of cotton or other natural or synthetic fabrics) by contacting the object with an aqueous solution of a detergent composition comprising the enzyme containing granule of the invention. Other processes for treatment of textile or cellulose containing fabric wherein the granule of the invention is applicable are desizing (preferably using amylase, lipase and/or protease enzymes), stone washing (preferably using glucanases enzymes such as cellulases), bleaching and/or coloring of textile (preferably using oxidoreductase enzymes).

Also due to process of the invention enabling improved control of granule size, low size granule, e.g. having an mean size between 100-400 $\mu$m, may be obtained directly from the mixer granulation process with reduced need for screening and recycling of odd sized granules. The process of the invention thus improves production economy considerably when producing low size granules. Low size granules are particularly useful in products such as animal feed/fodder compositions or baking flour compositions in which the granule will have an good mixability.

Especially for incorporation of enzyme granules in baking flour compositions, which usually are very fine powders the size of an enzyme granule is important. Also the protective nature of the granule will indeed protect the enzyme during a pelletizing process in the production of animal feed/fodder. Accordingly the invention provides a feed composition comprising the granule of the invention, a bakers flour composition comprising the granule of the invention and a method of preparing a bakers dough comprising the step of contacting the bakers flour composition comprising the enzyme granule with an aqueous liquid.

Further low size enzyme granules are useful for incorporation in liquid detergents as they due to their reduced size may form a stabile slurry in a liquid detergent.

Accordingly in a particular embodiment the invention encompasses a liquid detergent composition comprising the granule of the invention, preferably containing less than 10% water, more preferably less than 5%, most preferably less than 1% al percentages by weight.

For use in detergents the most common enzymes are proteases, amylases (e.g. $\alpha$- amylases) , cellulases, lipases and oxidoreductases. For use in baking compositions the most common enzymes are amyloglucosidases (glucoamylases, glucan 1, 4- $\alpha$- glucosidases) , bacterial $\alpha$- amylases, fungal $\alpha$- amylases, maltogenic amylases, glucose oxidases, proteases, pentosanases. For use in feed compositions the most common enzymes are bacterial $\alpha$- amylases, proteases, xylanases, phytases, while for use in textile treatment applications the most common enzymes are cellulases, $\alpha$- amylases.

## Detergent disclosure

A detergent composition of the invention comprises the enzyme containing granule of the invention and a surfactant. Additionally, it may optionally comprise a builder, another enzyme, a suds suppresser, a softening agent, a dye-transfer inhibiting agent and other components conventionally used in detergents such as soil-suspending agents, soil-releasing agents, optical brighteners, abrasives, bactericides, tarnish inhibitors, coloring agents, and/or encapsulated or non-encapsulated perfumes.

The detergent composition according to the invention can be in bars or granular forms or in liquid form. The pH (measured in aqueous solution at use concentration) will usually be neutral or alkaline, e.g. in the range of 7-11.

An enzyme contained in the granule of the invention incorporated in the detergent composition, is normally incorporated in the detergent composition at a level from 0.00001% to 2% of enzyme protein by weight of the composition, preferably at a level from 0.0001% to 1% of enzyme protein by weight of the composition, more preferably at a level from 0.001% to 0.5% of enzyme protein by weight of the composition, even more preferably at a level from 0.01% to 0.2% of enzyme protein by weight of the composition.

<u>Surfactant system</u>

The surfactant system may comprise nonionic, anionic, cationic, ampholytic, and/or zwitterionic surfactants. The surfactant system preferably consists of anionic surfactant or a combination of anionic and nonionic surfactant, e.g. 50-100 % of anionic surfactant and 0-50 % nonionic. The laundry detergent compositions may also contain cationic, ampholytic, zwitterionic, and semi-polar surfactants, as well as the nonionic and/or anionic surfactants other than those already described herein.

The surfactant is typically present at a level from 0.1% to 60% by weight. Some examples of surfactants are described below.

a) Nonionic surfactant:

The surfactant may comprise polyalkylene oxide (e.g. polyethylene oxide) condensates of alkyl phenols. The alkyl group may contain from about 6 to about 14 carbon atoms, in a straight chain or branched-chain. The ethylene oxide may be present in an amount equal to from about 2 to about 25 moles per mole of alkyl phenol.

The surfactant may also comprise condensation products of primary and secondary aliphatic alcohols with about 1 to about 25 moles of ethylene oxide. The alkyl chain of the aliphatic alcohol can either be straight or branched, and generally contains from about 8 to about 22 carbon atoms.

Further, the nonionic surfactant may comprise polyethylene oxide condensates of alkyl phenols, condensation products of primary and secondary aliphatic alcohols with from about 1 to about 25 moles of ethylene oxide, alkylpolysaccharides, and mixtures hereof. Most preferred are C8- C14 alkyl phenol ethoxylates having from 3 to 15 ethoxy groups and C8- C18 alcohol ethoxylates (preferably C10 avg.) having from 2 to 10 ethoxy groups, and mixtures thereof.

b) Anionic surfactants:

Suitable anionic surfactants include the alkyl sulfate surfactants which are water soluble salts or acids of the formula ROSO3M wherein R preferably is a C10-C24 hydrocarbyl, preferably an alkyl or hydroxyalkyl having a C10-C20 alkyl component, more preferably a C12-C18 alkyl or hydroxyalkyl, and M is H or a cation, e.g., an alkali metal cation (e.g. sodium, potassium, lithium), or ammonium or substituted ammonium.

Other anionic surfactants include salts (including, for example, sodium, potassium, ammonium, and substituted ammonium salts such as mono- di- and triethanolamine salts) of soap, C8-C22 primary or secondary alkanesulfonates, C8-$C_{24}$ olefinsulfonates, sulfonated polycarboxylic acids prepared by sulfonation of the pyrolyzed product of alkaline earth metal citrates.

Alkylbenzene sulfonates are suitable, especially linear (straight- chain) alkyl benzene sulfonates (LAS) wherein the alkyl group preferably contains from 10 to 18 carbon atoms. The laundry detergent compositions typically comprise from about 1% to about 40%, preferably from about 3% to about 20% by weight of such anionic surfactants.

<u>Builder system</u>

The compositions according to the present invention may further comprise a builder system. Any conventional builder system is suitable for use herein including aluminosilicate materials, silicates, polycarboxylates and fatty acids, materials such as ethylenediamine tetraacetate (EDTA), metal ion sequestrants such as aminopolyphosphonates. Phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.

Detergency builder salts are normally included in amounts of from 5% to 80% by weight of the composition. Preferred levels of builder for liquid detergents are from 5% to 30%.

<u>Bleaching agents</u>

The detergent composition may also comprise a bleaching agents, e.g. an oxygen bleach or a halogen bleach. The oxygen bleach may be a hydrogen peroxide releasing agent such as a perborate (e.g. PB1 or PB4) or a percarbonate, or it may e.g. be a percarboxylic acid. The particle size of a bleaching agent may be 400-800 microns. When present, oxygen bleaching compounds will typically be present at levels of from about 1% to about 25%.

The hydrogen peroxide releasing agent can be used in combination with bleach activators such as tetraacetylethylenediamine (TAED) , nonanoyloxybenzene- sulfonate (NOBS) , 3, 5- trimethyl- hexsanoloxybenzene- sulfonate (ISONOBS) or pentaacetylglucose (PAG) .

The halogen bleach may be, e.g. a hypohalite bleaching agent, for example, trichloro-isocyanuric acid and the sodium and potassium salt of dichloroisocyanurates and N-chloro and N-bromo alkane sulphonamides. Such materials are normally added at 0.5-10% by weight of the finished product, preferably 1-5% by weight.

Granular detergent compositions according to the present invention can also be in "compact form", i.e. they may have a relatively higher density than conventional granular detergents, i.e. form 550 to 950 g/l.

The compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and dishwashing operations.

More specifically, the enzyme containing granules of the invention may be incorporated in the detergent compositions described in WO 97/04079, WO 97/07202, WO 97/41212, and PCT/DK 97/00345.

## EXAMPLES

The present invention is further illustrated by the working examples described below, which are representative and not intended to be limiting. One skilled in the art will be capable of selecting other enzymes and particulate additives or methods on the basis of the teaching herein.

### EXAMPLE 1:

Preparation of mixer granulate comprising 15% wt. particulate Farigel

In a 50 litre Lödige mixer 14.1 kg of a powder composition prepared from the following:

1.8 kg of fibrous cellulose (Arbocel™ BFC200),
2.25 kg pre gelatinized wheat flour (Farigel de Ble F1100, WestHove, France)
0.9 kg of carbohydrate binder (Glucidex 21D, Roquette Freres)
0.6 kg of kaolin (Speswhite™, English China Clay) and
8.55 kg of finely ground sodium sulfate

were sprayed with a mixture of 2.5 kg of water and 0.9 kg of carbohydrate binder (Glucidex 21) and granulated and fluid bed dried as described in Example 1 in US 4,106,991.

The dry granulate was sieved to examine the size distribution of the granules.

### EXAMPLE 2:

Preparation of a granulate comprising 30% wt. particulate Farigel

In a 50 litre Lödige mixer 14.1 kg of a powder composition prepared from the following:

1.8 kg of fibrous cellulose (Arbocel™ BFC200),
4.5 kg pre gelatinized wheat flour (Farigel de Ble F1100, WestHove, France)
0.9 kg of carbohydrate binder (Glucidex 21D, Roquette Freres)
0.6 kg of kaolin (Speswhite™, English China Clay) and
6.3 kg of finely ground sodium sulfate

were sprayed with a mixture of 3.0 kg of water and 0.9 kg of carbohydrate binder (Glucidex 21) and granulated and fluid bed dried as described in Example 1 in US 4,106,991.

The dry granulate was sieved to examine the size distribution of the granules.

### EXAMPLE 3:

Preparation of granulate comprising 45% wt. particulate Farigel

In a 50 litre Lödige mixer 14.1 kg of a powder composition prepared from the following:

1.8 kg of fibrous cellulose (Arbocel™ BFC200),
6.75 kg pre gelatinized wheat flour (Farigel de Ble F1100, WestHove)
0.9 kg of carbohydrate binder (Glucidex 21D, Roquette Freres)
0.6 kg of kaolin (Speswhite™, English China Clay) and
4.05 kg of finely ground sodium sulfate

were sprayed with a mixture of 3.0 kg of water and 0.9 kg of carbohydrate binder (Glucidex 21) and granulated and fluid bed dried as described in Example 1 in US 4,106,991.

The dry granulate was sieved to examine the size distribution of the granules.

## EXAMPLE 4:

Preparation of granulate comprising 72% wt. particulate Farigel

In a 50 litre Lödige mixer 14.1 kg of a powder composition prepared from the following:

1.8 kg of fibrous cellulose (Arbocel™ BFC200),
10.8 kg pre gelatinized wheat flour (Farigel de Ble F1100, WestHove)
0.9 kg of carbohydrate binder (Glucidex 21D, Roquette Freres)
0.6 kg of kaolin (Speswhite™, English China Clay) and

were sprayed with a mixture of 3.0 kg of water and 0.9 kg of carbohydrate binder (Glucidex 21) and granulated and fluid bed dried as described in Example 1 in US 4,106,991.

The dry granulate was sieved to examine the size distribution of the granules.

## EXAMPLES 1-4: Particle Size Distribution

|  | EXAMPLE 1 15% Farigel % w/w | EXAMPLE 2 30% Farigel % w/w | EXAMPLE 3 45% Farigel % w/w | EXAMPLE 4 72% Farigel % w/w |
|---|---|---|---|---|
| > 500 $\mu$m | 39.2 | 26.1 | 20.1 | 25.9 |
| > 425 $\mu$m | 46.4 | 30.0 | 23.3 | 28.8 |
| > 355 $\mu$m | 53.6 | 34.6 | 26.8 | 30.3 |
| > 300 $\mu$m | 64.5 | 42.5 | 32.1 | 34.3 |
| > 212 $\mu$m | 89.7 | 80.1 | 60.8 | 40.6 |
| > 125 $\mu$m | 100.0 | 98.3 | 96.1 | 93.8 |
| < 125 $\mu$m | 0.0 | 1.7 | 4.9 | 6.2 |

The results show that with and increasing amount of particulate component (farigel) the mean particle size consistently drops from app. 400 $\mu$m to app. 200 $\mu$m with increased level of Farigel.

## EXAMPLES 5-7:

Three identical granules comprising 30 % wt. particulate Farigel were produced in order to test reproducibility.

In a 50 litre Lödige mixer 14.1 kg of a powder composition prepared from the following:

1.8 kg of fibrous cellulose (Arbocel™ BFC200),
4.5 kg pre gelatinized wheat flour (Farigel de Ble F1100, WestHove)
0.9 kg of carbohydrate binder (Glucidex 21D, Roquette Freres)
0.6 kg of kaolin (Speswhite™, English China Clay) and
6.3 kg of finely ground sodium sulfate

were sprayed with a mixture of 3.0 kg of water and 0.9 kg of carbohydrate binder (Glucidex 21) and granulated and fluid bed dried as described in Example 1 in US 4,106,991.

The dry granulate was sieved to examine the size distribution of the granules.

### EXAMPLE 8:

Preparation of granulate in larger scale comprising 30% particulate Farigel

In a 130 litre Lödige mixer 37.6 kg of a powder composition prepared from the following:

4.8 kg of fibrous cellulose (Arbocel™ BFC200),
12.0 kg pre gelatinized wheat flour (Farigel de Ble F1100, WestHove)
2.4 kg of carbohydrate binder (Glucidex 21D, Roquette Freres)
1.6 kg of kaolin (Speswhite™, English China Clay) and
16.8 kg of finely ground sodium sulfate

were sprayed with a mixture of 6.8 kg of water and 2.4 kg of carbohydrate binder (Glucidex 21) and granulated and fluid bed dried as described in Example 1 in US 4,106,991.

The dry granulate was sieved to examine the size distribution of the granules.

### EXAMPLES 5-8: Particle Size Distribution

|  | EXAMPLE 5<br>50 l Lödige % w/w | EXAMPLE 6<br>50 l Lödige % w/w | EXAMPLE 7<br>50 l Lödige % w/w | EXAMPLE 8<br>130 l Lödige % w/w |
|---|---|---|---|---|
| > 707 μm | 17.9 | 16.5 | 13.8 | 16.4 |
| > 600 μm | 24.6 | 22.4 | 19.1 | 21.1 |
| > 500 μm | 30.7 | 28.1 | 24.1 | 25.3 |
| > 425 μm | 35.8 | 32.6 | 28.0 | 28.4 |
| > 355 μm | 40.5 | 36.7 | 32.0 | 31.3 |
| > 300 μm | 45.9 | 41.7 | 37.0 | 34.3 |
| > 212 μm | 74.2 | 68.9 | 66.7 | 55.4 |
| > 125 μm | 98.5 | 97.7 | 98.9 | 93.4 |
| < 125 μm | 1.4 | 2.3 | 1.1 | 6.6 |

The results shows that the process is reproducible emphasizing the effect of the particulate component.

### EXAMPLE 9:

Preparation of standard phytase granulate

In a 50 litre Lödige mixer 14.4 kg of a powder composition prepared from the following:

1.8 kg of fibrous cellulose (Arbocel™ BFC200),
0.9 kg of carbohydrate binder (Glucidex 21D, Roquette Freres)
0.6 kg of kaolin (Speswhite™, English China Clay) and
11.1 kg of finely ground sodium sulfate

were sprayed with a mixture of 0.85 kg of phytase concentrate (Novo Nordisk A/S, Denmark) with 23% wt. dry matter, 2.25 kg of water and 0.3 kg of corn steep liquor and granulated and fluid bed dried as described in Example 1 in US 4,106,991.

The dry granulate was sieved to examine the size distribution of the granules.

**EXAMPLE 10:**

Preparation of phytase granulate comprising particulate Farigel

In a 50 litre Lödige mixer 14.4 kg of a powder composition prepared from the following:

1.8 kg of fibrous cellulose (Arbocel™ BFC200)
4.5 kg pre gelatinized wheat flour (Farigel de Ble F1100, WestHove)
0.9 kg of carbohydrate binder (Glucidex 21D, Roquette Freres)
0.6 kg of kaolin (Speswhite™, English China Clay) and
6.6 kg of finely ground sodium sulfate

were sprayed with a mixture of 0.85 kg of phytase concentrate (Novo Nordisk A/S, Denmark) with 23% wt. dry matter, 2.25 kg of water, and 0.3 kg of corn steep liquor and granulated and fluid bed dried as described in Example 1 in US 4,106,991.
The dry granulate was sieved to examine the size distribution of the granules.

**EXAMPLE 11:**

Preparation of standard phytase granulate comprising particulate Manna grits

In a 50 litre Lödige mixer 14.5 kg of a powder composition prepared from the following:

0.9 kg of fibrous cellulose (Arbocel™ BFC200)
7.5 kg pre Manna grits (In Danish: Mannagryn) (Particulate wheat, Havnemoellerne A/S, Denmark)
0.3 kg of carbohydrate binder (Glucidex 21D, Roquette Freres)
0.3 kg of kaolin (Speswhite™, English China Clay) and
5.5 kg of finely ground sodium sulfate

were sprayed with a mixture of 0.71 kg of phytase concentrate (Novo Nordisk A/S, Denmark) with 23% wt. dry matter, 1.80 kg of water, and 0.3 kg of corn steep liquor and granulated and fluid bed dried as described in Example 1 in US 4,106,991.
The dry granulate was sieved to examine the size distribution of the granules.

**EXAMPLES 9-11: Particle Size Distribution**

| | EXAMPLE 9 reference % w/w | EXAMPLE 10 Farigel % w/w | EXAMPLE 11 Manna grits % w/w |
|---|---|---|---|
| > 1200 μm | 19.1 | 6.1 | 2.2 |
| > 1000 μm | 25.2 | 8.9 | 2.4 |
| > 850 μm | 36.1 | 16.1 | 2.7 |
| > 710 μm | 50.1 | 25.2 | 3.6 |
| > 600 μm | 70.0 | 39.0 | 5.3 |
| > 500 μm | 84.1 | 49.4 | 28.9 |
| > 425 μm | 90.2 | 59.2 | 58.8 |
| > 355 μm | 94.2 | 64.9 | 80.6 |
| > 300 μm | 96.2 | 68.4 | 92.7 |
| < 250 μm | 0.6 | 22.7 | 6.2 |

The results shows that addition of particulate Farigel or Manna grits significantly reduces the mean particle size and results in a product suitable application in the animal feed industry. Further addition of Manna grits provides a narrow particle size distribution which is desirable.

**EXAMPLE 12:**

Preparation of Novamyl (an amylase) granulate comprising particulate Farigel

In a 50 liter Lödige mixer 12.75 kg of a powder composition prepared from the following:

1.8 kg of fibrous cellulose (Arbocel™ BFC200),
9.0 kg pre gelatinized wheat flour (Farigel de Ble F1100, WestHove)
1.35 kg of carbohydrate binder (Glucidex 21D, Roquette Freres)
0.6 kg of kaolin (Speswhite™, English China Clay) and

were sprayed with a 7.5 kg of Novamyl (Novo Nordisk A/S, Denmark) concentrate with 30% wt. dry matter and granulated and fluid bed dried as described in Example 1 in US 4,106,991.
The dry granulate was sieved to examine the size distribution of the granules.

**EXAMPLE 13:**

Preparation of Novamyl granulate comprising particulate Farigel

In a 50 litre Lödige mixer 12.75 kg of a powder composition prepared from the following:

1.8 kg of fibrous cellulose (Arbocel™ BFC200),
4.5 kg pre gelatinized wheat flour (Farigel de Ble F1100, WestHove)
1.35 kg of carbohydrate binder (Glucidex 21D, Roquette Freres)
0.6 kg of kaolin (Speswhite™, English China Clay) and
4.5 kg of finely ground sodium sulfate

were sprayed with a 7.5 kg of Novamyl (Novo Nordisk A/S, Denmark) concentrate with 30% dry matter and granulated and fluid bed dried as described in Example 1 in US 4,106,991.
The dry granulate was sieved to examine the size distribution of the granules.

**EXAMPLES 12-13: Particle Size Distribution**

|  | EXAMPLE 12 % w/w | EXAMPLE 13 % w/w |
|---|---|---|
| > 355 $\mu$m | 20.0 | 26.5 |
| > 300 $\mu$m | 22.6 | 30.7 |
| > 250 $\mu$m | 27.4 |  |
| > 180 $\mu$m | 58.0 | 39.8 |
| < 80 $\mu$m | 0.0 | 0.7 |

The results show that the addition of particulate Farigel results in a particle size distribution of the granules which is suitable for application to bakers flour.

**Claims**

1. A process for manufacture of a dry enzyme-containing granule comprising the step of adding a cereal grain flour to a mixer granulation process, wherein the cereal grain flour constitutes less than 75 of 100 parts of the finished granule and the cereal grain flour consists of particles having a mean size in their longest dimension which is at

least 40 μm and is less than half the diameter of the final granule.

2.	The process of claim 1, wherein the finished granule contains 3-15 particles.

3.	The process of claim 1 or 2, wherein the cereal grain is selected from wheat, rye, barley, oats, rice, maize or sorghum.

4.	The process of claim 3 wherein the cereal is wheat.

5.	The process of any preceding claim wherein the vegetable flour has been treated with dry superheated steam.

6.	The process of any preceding claim, wherein the enzyme is selected from hydrolases (EC 3.-.-.-).

7.	The process of claim 6 wherein the enzyme is selected from phytases (EC 3.1.3.-) and glycosidases (EC 3.2.-.-).

8.	The process of any preceding claim, wherein the granule further contains one or more granulating agents selected from fiber materials, binders, fillers, liquid agents, enzyme stabilizers, suspension agents, crosslinking agents, mediators and/or solvents.

9.	The process of claim 8, wherein the filler comprises finely ground alkali sulphate.

10.	The process of claim 8 or 9, wherein the filler comprises a clay.

11.	The process of any of claims 8-10, wherein the binder comprises a carbohydrate binder.

12.	The process of any of claims 8-11, wherein the fiber material comprises cellulose in fibrous form.

13.	The process of any preceding claim, further comprising a step of adding water, and a subsequent step of drying.

14.	The process of any preceding claim, wherein the finished granule comprises cellulose fibers and has a mean size below 480 μm.

15.	A detergent composition comprising a surfactant and granules obtainable from the process of any preceding claim.

16.	The detergent composition of claim 15 selected from dry compositions and liquid compositions containing less than 10 % w/w of water.

17.	An animal feed composition comprising granules obtainable from the process of any of claims 1-14.

18.	A bakers flour composition comprising granules obtainable from the process of any of claims 1-14.

19.	A method of preparing a dough comprising the step of contacting the bakers flour composition of claim 18 with an aqueous liquid.


**Patentansprüche**

1.	Verfahren zur Herstellung eines trockenen enzymenthaltenden Körnchens, umfassend den Schritt des Hinzufügens eines Getreidekornmehls zu einem Mischergranulationsverfahren, wobei das Getreidekornmehl weniger als 75 bis 100 Teile des fertigen Körnchens ausmachen und das Getreidekornmehl aus Partikeln besteht, die eine mittlere Größe in ihrer längsten Ausdehnung aufweisen, welche mindestens 40 μm beträgt und weniger als die Hälfte des Durchmessers des letztendlichen Körnchens beträgt.

2.	Verfahren nach Anspruch 1, wobei das fertige Körnchen 3-15 Partikel enthält.

3.	Verfahren nach Anspruch 1 oder 2, wobei das Getreidekorn ausgewählt ist aus Weizen, Roggen, Gerste, Hafer, Reis, Mais oder Sorghum.

4.	Verfahren nach Anspruch 3, wobei das Getreide Weizen ist.

5. Verfahren nach einem beliebigen vorhergehenden Anspruch, wobei das Pflanzenmehl mit trockenem überhitztem Dampf behandelt worden ist.

6. Verfahren nach einem beliebigen vorhergehenden Anspruch, wobei das Enzym ausgewählt ist aus Hydrolasen (EC 3.-.-.).

7. Verfahren nach Anspruch 6, wobei das Enzym ausgewählt ist aus Phytasen (EC 3.1.3.-) und Glykosidasen (EC 3.2.-.-).

8. Verfahren nach einem beliebigen vorhergehenden Anspruch, wobei das Körnchen des weiteren ein oder mehrere Granulierungsmittel enthält, ausgewählt aus Fasermaterialien, Bindern, Füllern, flüssigen Mitteln, Enzymstabilisatoren, Suspensionsmitteln, quervernetzenden Mitteln, Mediatoren und/oder Lösungsmitteln.

9. Verfahren nach Anspruch 8, wobei der Füller fein gemahlenes Alkalisulfat umfasst.

10. Verfahren nach Anspruch 8 oder 9, wobei der Füller eine Tonerde umfasst.

11. Verfahren nach einem beliebigen der Ansprüche 8-10, wobei der Binder einen Kohlenhydratbinder umfasst.

12. Verfahren nach einem beliebigen der Ansprüche 8-11, wobei das Fasermaterial Cellulose in fibröser Form umfasst.

13. Verfahren nach einem beliebigen vorhergehenden Anspruch, des weiteren umfassend einen Schritt des Hinzufügens von Wasser und einen anschließenden Trocknungsschritt.

14. Verfahren nach einem beliebigen vorhergehenden Anspruch, wobei das fertige Körnchen Cellulosefasern umfasst und eine mittlere Größe von unter 480 $\mu$m aufweist.

15. Detergenszusammensetzung, umfassend ein oberflächenaktives Mittel und Körnchen, die aus dem Verfahren gemäß einem beliebigen vorhergehenden Anspruch erhältlich sind.

16. Detergenszusammensetzung nach Anspruch 15, ausgewählt aus Trockenzusammensetzungen und Flüssigzusammensetzungen, die weniger als 10 % Gew./Gew. Wasser enthalten.

17. Tierfutterzusammensetzung, umfassend Körnchen, erhältlich nach dem Verfahren gemäß einem beliebigen der Ansprüche 1-14.

18. Backmehlzusammensetzung, umfassend Körnchen, erhältlich nach dem Verfahren gemäß einem beliebigen der Ansprüche 1-14.

19. Verfahren zum Herstellen eines Teiges, umfassend den Schritt des Inkontaktbringens der Backmehlzusammensetzung gemäß Anspruch 18 mit einer wässrigen Flüssigkeit.

## Revendications

1. Procédé de fabrication d'un granule sec contenant une enzyme comprenant l'étape d'ajout d'une farine de grains de céréale à un procédé de granulation dans un mélangeur, dans lequel la farine de grains de céréale représente moins de 75 de 100 parties du granule fini et la farine de grains de céréale est composée de particules ayant une taille moyenne dans leur dimension la plus longue qui est d'au moins 40 $\mu$m et inférieure à la moitié du diamètre du granule final.

2. Procédé selon la revendication 1, dans lequel le granule fini comprend de 3 à 15 particules.

3. Procédé selon la revendication 1 ou 2, dans lequel le grain de céréale est choisi parmi le blé, le seigle, l'orge, l'avoine, le riz, le maïs ou le sorgho.

4. Procédé selon la revendication 3, dans lequel la céréale est le blé.

5. Procédé selon l'une quelconque des revendications précédentes, dans lequel la farine végétale a été traitée avec une vapeur sèche superchauffée.

6. Procédé selon l'une quelconque des revendications précédentes, dans lequel l'enzyme est choisie parmi les hydrolases (EC 3.-.-.-).

7. Procédé selon la revendication 6, dans lequel l'enzyme est choisie parmi les phytases (EC 3.1.3.-) et les glycosidases (EC 3.2.-.-).

8. Procédé selon l'une quelconque des revendications précédentes, dans lequel le granule comprend en outre un ou plusieurs agents de granulation choisis parmi les matières fibreuses, les liants, les charges, les agents liquides, les stabilisateurs d'enzymes, les agents de suspension, les agents de réticulation, les médiateurs et/ou les solvants.

9. Procédé selon la revendication 8, dans lequel la charge comprend du sulfate alcalin finement broyé.

10. Procédé selon la revendication 8 ou 9, dans lequel la charge comprend une argile.

11. Procédé selon l'une quelconque des revendications 8 à 10, dans lequel le liant comprend un liant glucidique.

12. Procédé selon l'une quelconque des revendications 8 à 11, dans lequel la matière fibreuse comprend la cellulose sous une forme fibreuse.

13. Procédé selon l'une quelconque des revendications précédentes, comprenant en outre une étape d'ajout d'eau, puis une étape ultérieure de séchage.

14. Procédé selon l'une quelconque des revendications précédentes, dans lequel le granule fini comprend des fibres cellulosiques et a une taille moyenne inférieure à 480 $\mu$m.

15. Composition détergente comprenant un tensioactif et des granules susceptibles d'être obtenus par le procédé selon l'une quelconque des revendications précédentes.

16. Composition détergente selon la revendication 15, choisie parmi les compositions sèches et les compositions liquides contenant moins de 10 % m/m d'eau.

17. Composition alimentaire pour animaux comprenant les granules susceptibles d'être obtenus par le procédé selon l'une quelconque des revendications 1 à 14.

18. Composition de farine de boulanger comprenant les granules susceptibles d'être obtenus par le procédé selon l'une quelconque des revendications 1 à 14.

19. Procédé de préparation d'une pâte comprenant l'étape de mise en contact de la composition de farine de boulanger selon la revendication 18 avec un liquide aqueux.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9723606 A
- WO 9739116 A
- EP 170360 B1
- EP 304332 B1
- EP 304331 A
- WO 9009440 A
- WO 9009428 A
- WO 9854980 A
- EP 321481 B1
- EP 257996 B1, CULTOR
- EP 168526 B1, HENKEL
- WO 9211347 A, HENKEL
- WO 9742837 A
- WO 9743482 A
- WO 9742839 A
- US 4106991 A
- EP 304331 B1
- EP 251446 A
- WO 9100345 A
- EP 525610 A
- WO 9402618 A
- WO 9606931 A
- US 4106991 E
- WO 8908694 A
- WO 8908695270608 B1
- DK PA199800876
- EP 170360 A
- EP 304332 A
- EP 458849 A
- EP 458845 A
- WO 9212645 A
- WO 8908695 A
- WO 8707292 A
- WO 9106638 A
- WO 9213030 A
- WO 9307260 A
- WO 9307263 A
- WO 9638527 A
- WO 9616151 A
- US 5324649 A
- US 4689297 A
- EP 206417 A
- EP 193829 A
- DE 4344215
- DE 4322229 A
- DD 263790
- JP 61162185 A
- JP 58179492 B
- WO 9704079 A
- WO 9707202 A
- WO 9741212 A
- DK 9700345 W

### Non-patent literature cited in the description

- Powdered detergents. Surfactant Science Series. Marcel Dekker, 1998, vol. 71, 140-142
- **HARNBY et al.** *Mixing in the Process Industries,* IS-BN 0-408-11574-2, 39-53
- Nomenclature Committee of the International Union of Biochemistry and Molecular Biology. Academic Press, Inc, 1992
- **HARNBY et al.** *Mixing in the Process Industries,* IS-BN 0-408-11574-2, 54-77